# EUROPEAN PATENT APPLICATION

(11) **EP 2 916 251 A1**
(43) Date of publication of application: **09.09.2015**
(21) Application number: 15157412.6
(22) Date of filing: 03.03.2015
(51) Int. Cl.: G06F 19/00, G06Q 50/24, H04L 12/58, H04L 29/08

(54) **METHOD AND SYSTEM FOR CONTEXT-DRIVEN REAL-TIME MESSAGING OF HEALTHCARE INFORMATION**

(30) Priority: 04.03.2014 US 201414196001
(71) Applicant: Siemens Medical Solutions USA, Inc., Malvern, PA 19355-1406 (US)
(72) Inventor: Shrestha, Sumit, Malvern, PA 19355 (US); Manioti, Evangelia, Harleysville, PA 19438 (US); Lutz, Michael H., Wayne, PA 19087 (US); Parisi, Eduardo, Downingtown, PA 19335 (US); Dolphin, Edward, Wilmington, DE 19810 (US)
(74) Representative: Wallin, Nicholas James

(57) **Abstract**

A computer implemented communication method between healthcare professionals. includes receiving, from a first electronic device, a text communication having a patient identifier corresponding to a patient and identifying, via a computer, the patient identifier of the text communication. The method also includes retrieving, via the computer, one or more portions of electronic patient medical records corresponding to the patient identifier and transmitting, via a real time messaging server, the text communication and the one or more portions of the electronic patient medical records to a second electronic device. The method further includes simultaneously displaying the text communication and the one or more portions of the electronic patient medical records at the second electronic device.

## Description

### TECHNOLOGY FIELD

The present application relates generally to methods, systems, and apparatuses for combining real time text communication methods with access to an electronic medical record (EMR) database, and in particular, to methods, systems, and apparatuses for displaying, on electronic devices, real time text communications and medical records of a patient from an EMR database.

### BACKGROUND

Healthcare professionals are under pressure to deliver the highest level of care to their patients. Clinicians (e.g., doctors, nurses and staff personnel) rely heavily on electronic medical records to connect themselves with patient data and each other to improve patient care management. Many clinicians practice in large care centers with multiple locations, which can make it difficult for them to discuss patient care issues in person or over the phone.

Clinicians are often asked to immediately or quickly address patient needs. At any given time, however, a clinician, such as a doctor, may be at a different location than another clinician who initially receives information from a patient, such a nurse or staff. For example, a doctor may be at a hospital when a patient calls or visits the doctor's office for immediate attention. Accordingly, these needs are typically addressed by electronic communication (e.g., telephone communication, voice messages, and e-mails) between clinicians.

### SUMMARY

Embodiments of the present invention include a computer implemented communication method between healthcare professionals. The method includes receiving, from a first electronic device, a text communication having a patient identifier corresponding to a patient and identifying, via a computer, the patient identifier of the text communication. The method also includes retrieving, via the computer, one or more portions of electronic patient medical records corresponding to the patient identifier and transmitting, via a real time messaging server, the text communication and the one or more portions of the electronic patient medical records to a second electronic device. The method further includes simultaneously displaying the text communication and the one or more portions of the electronic patient medical records at the second electronic device.

According to one embodiment, the method further includes simultaneously displaying the text communication and the one or more portions of the electronic patient medical records at the first electronic device.

According to another embodiment, the method further includes receiving a response text communication from the second electronic device and simultaneously displaying the response text communication and the one or more portions of electronic patient medical records at the first electronic device and the second electronic device.

In one embodiment, the text communication or the response text communication comprises a medical record identifier and the method further comprises simultaneously displaying the text communication or the response text communication and one or more additional portions of the electronic patient medical records corresponding to the medical record identifier.

In another embodiment, the medical record identifier in the text communication or the response text communication is a part of a patient's anatomy and the method further comprises simultaneously displaying the text communication or the response text communication and the one or more additional portions of the electronic patient medical records corresponding to the part of the patient's anatomy.

According to one embodiment, the medical record identifier in the text communication or the response text communication is a current treatment of the patient and the method further comprises simultaneously displaying the text communication or the response text communication and the one or more additional portions of the electronic patient medical records corresponding to the current treatment of the patient

In one aspect of an embodiment, the medical record identifier in the text communication or the response text communication comprises one or more medications prescribed to the patient and the method further comprises simultaneously displaying the text communication or the response text communication and the one or more additional portions of the electronic patient medical records corresponding to the one or more medications prescribed to the patient.

According to one embodiment, simultaneously displaying the text communication and the one or more portions of the electronic patient medical records further includes displaying the text communication in a first display area of a display and displaying the one or more portions of the electronic patient medical records in a second display area of the display.

According to one embodiment, the method further includes identifying a plurality of patients each having one or more portions of electronic patient medical records that correspond to the patient identifier and displaying the identification of the plurality of patients having one or more portions of electronic patient medical records and receiving, from the first electronic device, a second text communication having a second patient identifier corresponding to the patient, the second patient identifier being different from the first patient identifier.

Embodiments of the present invention include a healthcare professional network communication system that includes a server and a first presentation module. The server includes: (i) a receiving module configured to receive a text communication from a first electronic device, the text communication having a patient identifier corresponding to a patient; (ii) a database interface module configured to retrieve one or more portions of electronic patient medical records corresponding to the patient identifier; and (iii) a transmitting module configured to transmit, via a real time messaging server, the text communication and the one or more portions of electronic patient medical records to a second electronic device. The first presentation module is located at the second electronic device and is configured to simultaneously present the text communication and the one or more portions of the electronic patient medical records on a display of the second electronic device.

According to one embodiment, the receiving module is further configured to receive a response text communication from the second electronic device; and the system further includes a second presentation module at the first electronic device configured to present at least one of the text communication and the response text communication simultaneously with the one or more portions of the electronic patient medical records on a display of the first electronic device.

According to another embodiment, the text communication or the response text communication includes a medical record identifier and the database interface module is further configured to retrieve one or more additional portions of the electronic patient medical records corresponding to the medical record identifier. The transmitting module is further configured to transmit, via the real time messaging server, the text communication or the response text communication and the one or more additional portions of the electronic patient medical records between the first electronic device and the second electronic device. The first presentation module at the second electronic device is further configured to present the text communication or the response text communication simultaneously with the one or more additional portions of the electronic patient medical records on the display of the second electronic device. The second presentation module at the first electronic device is further configured to present the text communication or the response text communication simultaneously with the one or more additional portions of the electronic patient medical records on the display of the first electronic device.

In one embodiment, the first presentation module at the second electronic device is further configured to simultaneously present: (i) the text communication at a first area of the display at the second electronic device; and (ii) the one or more portions of the electronic patient medical records at a second area of the display at the second electronic device

In one aspect of an embodiment, the database interface module is further configured to identify a plurality of patients each having one or more portions of electronic patient medical records that correspond to the patient identifier. The first presentation module is further configured to display the identification of the plurality of patients each having one or more portions of electronic patient medical records. The receiving module is further configured to receive a second text communication from the first electronic device having a second patient identifier corresponding to the patient, the second patient identifier being different from the first patient identifier. The database interface module is configured to retrieve the one or more portions of the electronic patient medical records corresponding to the second patient identifier.

Embodiments of the present invention include an article of manufacture for communicating between healthcare professionals. The article of manufacture includes a non-transitory, tangible computer-readable medium holding computer-executable instructions for performing a method that includes receiving, from a first electronic device, a text communication having a patient identifier corresponding to a patient and identifying, via a computer, the patient identifier of the communication. The method also includes retrieving, via the computer, one or more portions of electronic patient medical records corresponding to the patient identifier and transmitting, via a real time messaging server, the text communication and the electronic medical record data to a second electronic device. The method further includes simultaneously displaying the text communication and the one or more portions of the electronic patient medical records at the second electronic device.

According to one embodiment, the instructions further include simultaneously displaying the text communication and the one or more portions of the electronic patient medical records at the first electronic device.

According to one embodiment, the instructions further include receiving a response text communication from the second electronic device and simultaneously displaying the response text communication and the one or more portions of the electronic patient medical records at the first electronic device and the second electronic device.

In one embodiment, the text communication or the response text communication comprises a medical record identifier and further comprising instructions for simultaneously displaying the text communication or the response text communication and one or more additional portions of the electronic patient medical records corresponding to the medical record identifier.

In another embodiment, the instructions further include simultaneously displaying the text communication and the one or more portions of the electronic patient medical records further comprises displaying the text communication in a first display area of a display and displaying the one or more portions of the electronic patient medical records in a second display area of the display.

In yet another embodiment, the instructions further include identifying a plurality of patients each having one or more portions of electronic patient medical records that correspond to the patient identifier, displaying the identification of the plurality of patients having one or more portions of electronic patient medical records and receiving, from the first electronic device, a second text communication having a second patient identifier corresponding to the patient, the second patient identifier being different from the first patient identifier.

### BRIEF DESCRIPTION OF THE DRAWINGS

The foregoing and other aspects of the present invention are best understood from the following detailed description when read in connection with the accompanying drawings. For the purpose of illustrating the invention, there is shown in the drawings embodiments that are presently preferred, it being understood, however, that the invention is not limited to the specific instrumentalities disclosed. Included in the drawings are the following Figures:
FIG. 1 is a system block diagram illustrating a healthcare professional network communication system for use with embodiments disclosed herein;
FIG. 2 is a system diagram illustrating elements of components of the healthcare professional network communication system for use with embodiments disclosed herein;
FIG. 3 illustrates an example of a computing environment within which embodiments of the invention may be implemented; and
FIG. 4A through FIG. 4J are exemplary screen shots illustrating display of real time text communications and electronic patient medical records on an electronic device according to embodiments of the invention.

### DETAILED DESCRIPTION OF EXEMPLARY EMBODIMENTS

As described above, patient needs are typically addressed by electronic communication (e.g., telephone communication, voice messages and e-mails) between clinicians, such as doctors, at different locations from other clinicians, such as nurses or staff. Often, this communication results in waiting for one clinician to reply to another. For example, a nurse may leave a voice message or e-mail for a doctor and then wait for the doctor to reply.

Further, when the clinicians finally do communicate with each other, one clinician may not have access to the same data in the electronic medical record of the patient. For example, clinicians may use conventional real time text communication methods (e.g., instant messaging (IM), short message service (SMS)) to address a patient's needs. These conventional real time text communication methods do not, however, present electronic medical record data of the patient to the clinicians. Accordingly, time may be wasted in ascertaining information about the patient. For example, addressing a patient's needs through conventional text communication methods typically includes back and forth communications between clinicians about the patient (e.g., identification of the patient, previous diagnosis of the patient, current medications) that could otherwise be ascertained from the electronic medical records of a patient.

Embodiments include systems and methods for providing more efficient communication between clinicians. Embodiments combine real time text communication methods with access to an electronic medical record (EMR) database. Embodiments display text using real time text communication between users (e.g., clinicians) and electronic medical records from an electronic medical records database. Some embodiments display electronic medical records stored in an EMR database responsive to input using the real time text communication. Some embodiments provide simultaneous display of real time text communication and patient electronic medical records.

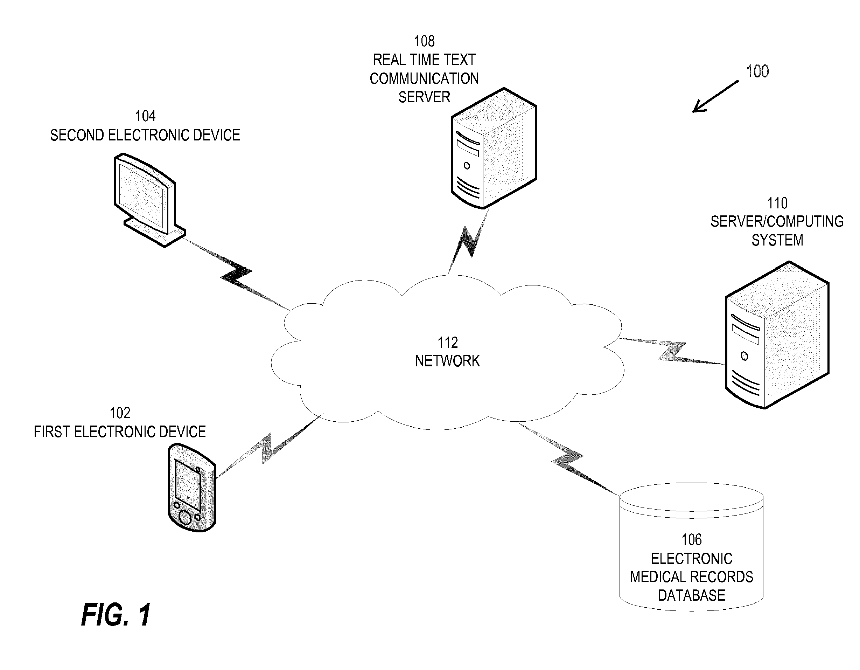

FIG. 1 is a system block diagram illustrating a healthcare professional network communication system for use with embodiments disclosed herein. As shown in FIG. 1, the system 100 may include a first electronic device 102, a second electronic device 104, an EMR database 106, a real time text communication server 108 and a server/computing system 110 that communicates via network 112.

First electronic device 102 and second electronic device 104 may include any electronic device configured to transmit, receive and display text and data (e.g., images). Examples of first electronic device 102 and second electronic device 104 include a personal computer (laptop or desktop), a network computer, and a mobile device (e.g., smart phone, personal digital assistant (PDA), a tablet computer). The exemplary system 100 shown in FIG. 1 includes two electronic devices 102 and 104. Other embodiments may, however, include any number of electronic devices configured to transmit, receive and display text and data over network 112.

An EMR database may be any medium that includes data collected over the course of one or more patients' treatments, and may draw from multiple data sources. An exemplary EMR database includes, for example, computed tomography (CT) images, X-ray images, laboratory test results, doctor progress notes, details about medical procedures, prescription drug information, radiological reports, other specialist reports, demographic information, and billing (financial) information. Structured data sources, such as financial, laboratory, and pharmacy databases, generally maintain patient information in database tables. Information may also be stored in unstructured data sources, such as free text, images, waveforms, or physician reports (e.g., dictations).

Real time text communication server 108 may include any server configured to manage/facilitate transmission of real time text communication (e.g., IM, SMS) between electronic devices across a network (e.g., network 112). The exemplary system 100 shown in FIG. 1 includes a single real time text communication server 108. Other embodiments may, however, include any number of real time text communication server configured to manage/facilitate transmission of real time text communication between any number of electronic devices.

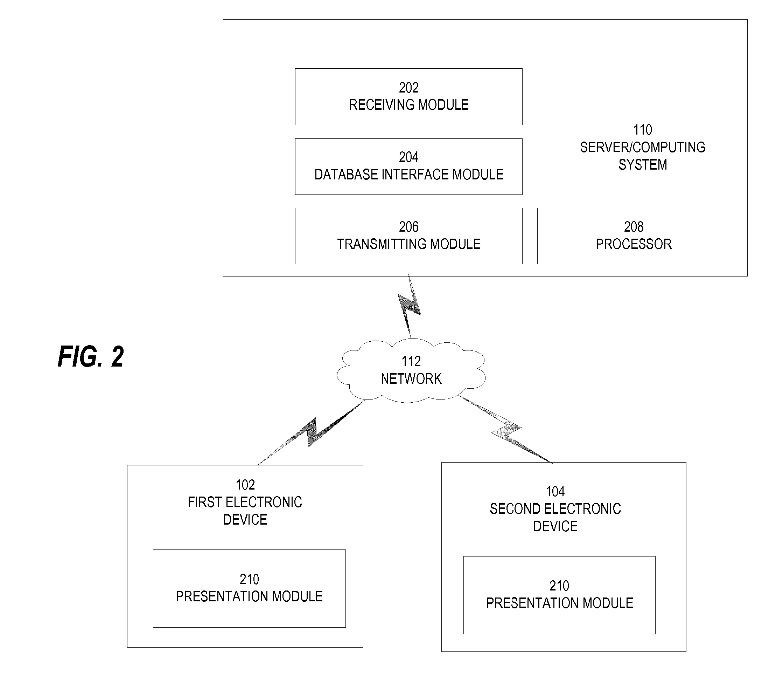

FIG. 2 is a system diagram illustrating elements of server/computing system 110, first electronic device 102 and second electronic device 104 of communication system 100 shown in FIG. 1 for use with embodiments disclosed herein. As shown in FIG. 2, server/computing system 110 may include receiving module 202, database interface module 204, transmitting module 206 and processor 208. In some embodiments, however, exemplary server/computing systems may include more than one processor.

Receiving module 202 may be configured to receive text communications from one or more electronic devices, such as first electronic device 102 and/or second electronic device 104. As described above, embodiments may include any number of electronic devices configured to transmit, receive and display text and data over network 112. Accordingly, in some embodiments, receiving module 202 may be configured to receive text communications from any number of electronic devices. For simplicity of explanation, however, the embodiments described herein include communications between first electronic device 102 and second electronic device 104.

Server/computing system 110 may identifying, via processor 208, a patient identifier of a text communication and database interface module 204 may be configured to retrieve one or more portions of electronic medical records of a patient corresponding to the patient identifier included in a text communication received at receiving module 202. For example, database interface module 204 may be configured to retrieve one or more portions of electronic medical records from EMR database 106, via processor 208, corresponding to a patient identifier included in the text communication received at receiving module 202.

Transmitting module 206 may be configured to transmit to second electronic device 104, via a real time messaging server 108 (shown in FIG. 1), the text communication received at receiving module 202 and the one or more portions of electronic medical records retrieved from EMR database 106.

First electronic device 102 and second electronic device 104 may each include a presentation module 210 configured to cause the text communications transmitted from transmitting module 206 and the electronic medical records retrieved from EMR database 106 to be simultaneously presented on a display of the second electronic device 104. The presentation module 210 may include any software used to simultaneously present text communications and electronic medical records on a display of an electronic device, including, but not limited to mobile device applications, web pages and desk top software.

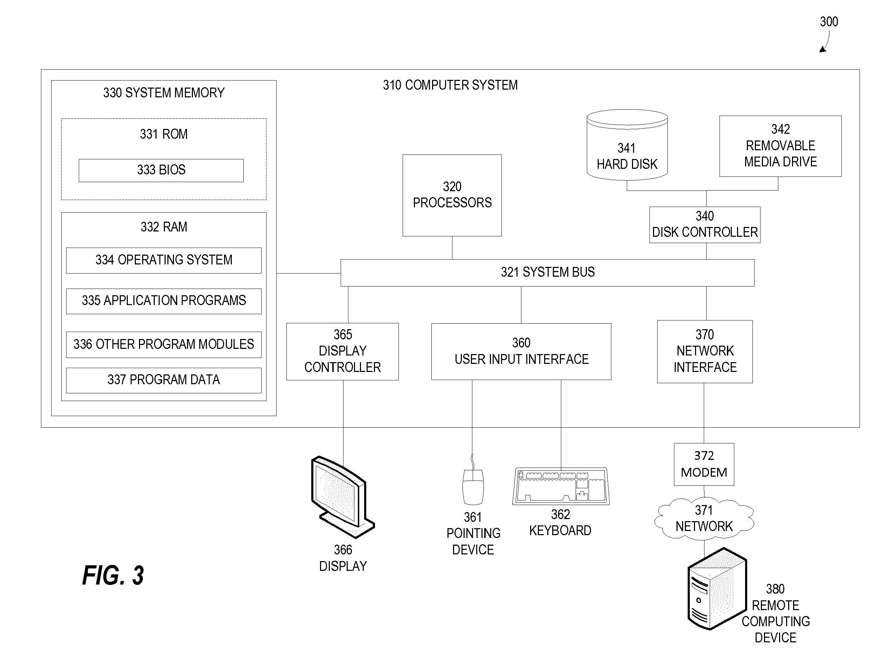

FIG. 3 illustrates an example of a computing environment 300 within which embodiments of the invention may be implemented. Computing environment 300 may be implemented as part of any component described herein, including for example, first electronic device 102, second electronic device 104, real time text communication server 108 and server/computing system 110. Computing environment 300 may include computer system 310, which is one example of a computing system upon which embodiments of the invention may be implemented. As shown in FIG. 3, the computer system 310 may include a communication mechanism such as a bus 321 or other communication mechanism for communicating information within the computer system 310. The system 310 further includes one or more processors 320 coupled with the bus 321 for processing the information. The processors 320 may include one or more CPUs, GPUs, or any other processor known in the art.

The computer system 310 also includes a system memory 330 coupled to the bus 321 for storing information and instructions to be executed by processors 320. The system memory 330 may include computer readable storage media in the form of volatile and/or nonvolatile memory, such as read only memory (ROM) 331 and/or random access memory (RAM) 332. The system memory RAM 332 may include other dynamic storage device(s) (e.g., dynamic RAM, static RAM, and synchronous DRAM). The system memory ROM 331 may include other static storage device(s) (e.g., programmable ROM, erasable PROM, and electrically erasable PROM). In addition, the system memory 330 may be used for storing temporary variables or other intermediate information during the execution of instructions by the processors 320. A basic input/output system 333 (BIOS) containing the basic routines that help to transfer information between elements within computer system 310, such as during start-up, may be stored in ROM 331. RAM 332 may contain data and/or program modules that are immediately accessible to and/or presently being operated on by the processors 320. System memory 330 may additionally include, for example, operating system 334, application programs 335, other program modules 336 and program data 337.

The computer system 310 also includes a disk controller 340 coupled to the bus 321 to control one or more storage devices for storing information and instructions, such as a magnetic hard disk 341 and a removable media drive 342 (*e.g*., floppy disk drive, compact disc drive, tape drive, and/or solid state drive). The storage devices may be added to the computer system 310 using an appropriate device interface (*e.g*., a small computer system interface (SCSI), integrated device electronics (IDE), Universal Serial Bus (USB), or FireWire).

The computer system 310 may also include a display controller 365 coupled to the bus 321 to control a display or monitor 366, such as a cathode ray tube (CRT) or liquid crystal display (LCD), for displaying information to a computer user. The computer system includes a user input interface 360 and one or more input devices, such as a keyboard 362 and a pointing device 361, for interacting with a computer user and providing information to the processor 320. The pointing device 361, for example, may be a mouse, a trackball, or a pointing stick for communicating direction information and command selections to the processor 320 and for controlling cursor movement on the display 366. The display 366 may provide a touch screen interface which allows input to supplement or replace the communication of direction information and command selections by the pointing device 361.

The computer system 310 may perform a portion or all of the processing steps of embodiments of the invention in response to the processors 320 executing one or more sequences of one or more instructions contained in a memory, such as the system memory 330. Such instructions may be read into the system memory 330 from another computer readable medium, such as a hard disk 341 or a removable media drive 342. The hard disk 341 may contain one or more datastores and data files used by embodiments of the present invention. Datastore contents and data files may be encrypted to improve security. The processors 320 may also be employed in a multi-processing arrangement to execute the one or more sequences of instructions contained in system memory 330. In alternative embodiments, hard-wired circuitry may be used in place of or in combination with software instructions. Thus, embodiments are not limited to any specific combination of hardware circuitry and software.

As stated above, the computer system 310 may include at least one computer readable medium or memory for holding instructions programmed according to embodiments of the invention and for containing data structures, tables, records, or other data described herein. The term "computer readable medium" as used herein refers to any non-transitory, tangible medium that participates in providing instructions to the processor 320 for execution. A computer readable medium may take many forms including, but not limited to, nonvolatile media, volatile media, and transmission media. Non-limiting examples of nonvolatile media include optical disks, solid state drives, magnetic disks, and magneto-optical disks, such as hard disk 341 or removable media drive 342. Non-limiting examples of volatile media include dynamic memory, such as system memory 330. Non-limiting examples of transmission media include coaxial cables, copper wire, and fiber optics, including the wires that make up the bus 321. Transmission media may also take the form of acoustic or light waves, such as those generated during radio wave and infrared data communications.

The computing environment 300 may further include the computer system 320 operating in a networked environment using logical connections to one or more remote computers, such as remote computer 380. Remote computer 380 may be a personal computer (laptop or desktop), a mobile device, a server, a router, a network PC, a peer device or other common network node, and typically includes many or all of the elements described above relative to computer 310. When used in a networking environment, computer 310 may include modem 372 for establishing communications over a network 371, such as the Internet. Modem 372 may be connected to system bus 321 via network interface 370, or via another appropriate mechanism.

Network 371 may be any network or system generally known in the art, including the Internet, an intranet, a local area network (LAN), a wide area network (WAN), a metropolitan area network (MAN), a direct connection or series of connections, a cellular telephone network, or any other network or medium capable of facilitating communication between computer system 310 and other computers (e.g., remote computing system 380). The network 371 may be wired, wireless or a combination thereof. Wired connections may be implemented using Ethernet, Universal Serial Bus (USB), RJ-11 or any other wired connection generally known in the art. Wireless connections may be implemented using Wi-Fi, WiMAX, and Bluetooth, infrared, cellular networks, satellite or any other wireless connection methodology generally known in the art. Additionally, several networks may work alone or in communication with each other to facilitate communication in the network 371.

An executable application, as used herein, comprises code or machine readable instructions for conditioning the processor to implement predetermined functions, such as those of an operating system, a context data acquisition system or other information processing system, for example, in response to user command or input. An executable procedure is a segment of code or machine readable instruction, sub-routine, or other distinct section of code or portion of an executable application for performing one or more particular processes. These processes may include receiving input data and/or parameters, performing operations on received input data and/or performing functions in response to received input parameters, and providing resulting output data and/or parameters. A graphical user interface (GUI), as used herein, comprises one or more display images, generated by a display processor and enabling user interaction with a processor or other device and associated data acquisition and processing functions.

The GUI also includes an executable procedure or executable application. The executable procedure or executable application conditions the display processor to generate signals representing the GUI display images. These signals are supplied to a display device which displays the image for viewing by the user. The executable procedure or executable application further receives signals from user input devices, such as a keyboard, mouse, light pen, touch screen or any other means allowing a user to provide data to a processor. The processor, under control of an executable procedure or executable application, manipulates the GUI display images in response to signals received from the input devices. In this way, the user interacts with the display image using the input devices, enabling user interaction with the processor or other device. The functions and process steps herein may be performed automatically or wholly or partially in response to user command. An activity (including a step) performed automatically is performed in response to executable instruction or device operation without user direct initiation of the activity.

The system and processes of the figures presented herein are not exclusive. Other systems, processes and menus may be derived in accordance with the principles of the invention to accomplish the same objectives. Although this invention has been described with reference to particular embodiments, it is to be understood that the embodiments and variations shown and described herein are for illustration purposes only. Modifications to the current design may be implemented by those skilled in the art, without departing from the scope of the invention. Further, the processes and applications may, in alternative embodiments, be located on one or more (e.g., distributed) processing devices on a network linking the units of FIG. 3. Any of the functions and steps provided in the Figures may be implemented in hardware, software or a combination of both. No claim element herein is to be construed under the provisions of 35 U.S.C. 112, sixth paragraph, unless the element is expressly recited using the phrase "means for."

The embodiments of the present disclosure may be implemented with any combination of hardware and software. In addition, the embodiments of the present disclosure may be included in an article of manufacture (e.g., one or more computer program products) having, for example, computer-readable, non-transitory media. The media has embodied therein, for instance, computer readable program code for providing and facilitating the mechanisms of the embodiments of the present disclosure. The article of manufacture can be included as part of a computer system or sold separately.

Exemplary embodiments of using the system 100 shown in FIG. 1 and the elements of the system 100 (at FIG. 1) that are shown in FIG. 2 are now described. Embodiments may include any number of electronic devices, including the display of real time text communication and electronic patient medical records simultaneously on any number of electronic devices. Embodiments described herein, however, include communications between first electronic device 102 and second electronic device 104 for simplicity of explanation.

Receiving module 202 of server 110 may receive a text communication from first electronic device 102. The text communication may include a patient identifier corresponding to a patient. For example, upon interaction with a patient at a hospital, a nurse may send the text communication from the first electronic device (e.g., desktop to a treating doctor of the patient. A patient identifier may be any text character or characters to identify a specific patient. Examples of patient identifiers include a first name of a patient, a last name of a patient, a full name of a patient and a numeric identifier (e.g., patient ID number, birthdate, social security number).

Server/computing system 110 may then identify the patient identifier of the text communication sent from first electronic device 102. When the patient identifier has been identified, database interface module 204 may be configured to retrieve one or more portions of electronic patient medical records corresponding to a patient identifier included in the text communication received at receiving module 202. For example, database interface module 204 may be configured to retrieve one or more portions of electronic patient medical records from EMR database 106 corresponding to a patient identifier included in the text communication received at receiving module 202.

Server/computing system 110 may then identify the location of second electronic device 104 using a destination identifier (e.g., IP address, mobile phone number) sent with the text communication and transmit, via a real time text communication server 108, the text communication and the electronic patient medical records to the second electronic device 102. The text communication and the electronic patient medical records may then be displayed, via presentation module 210, at the second electronic device 104.

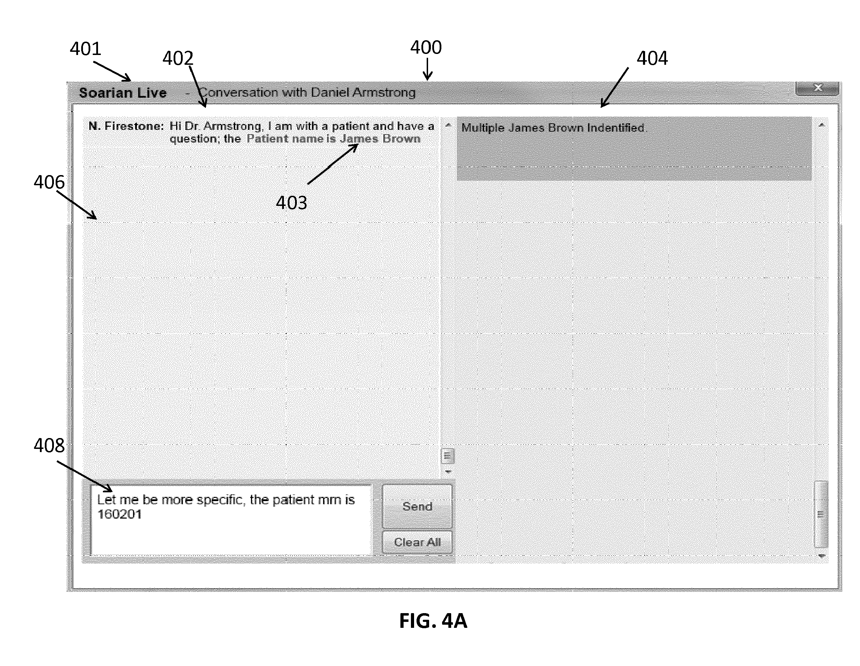

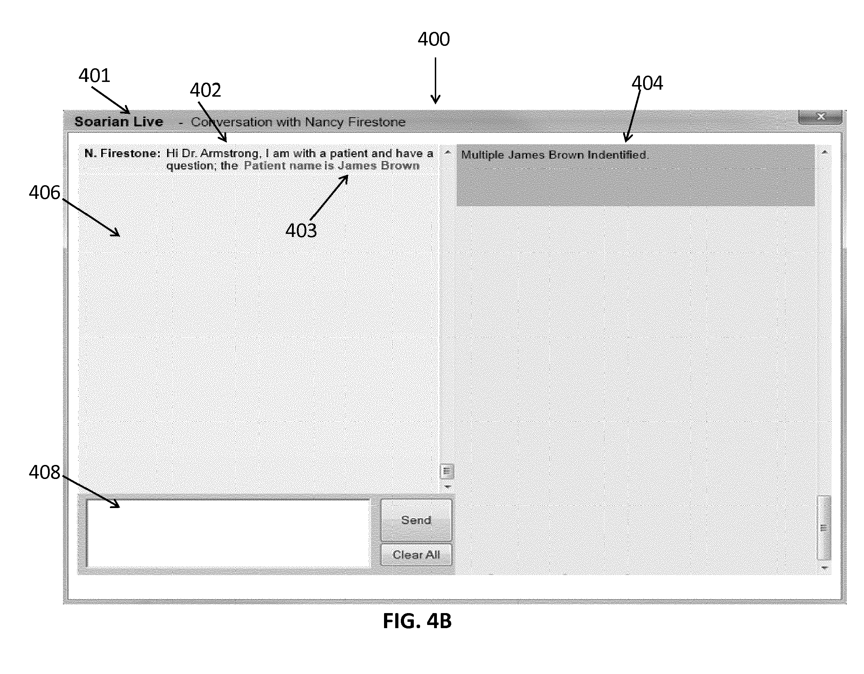

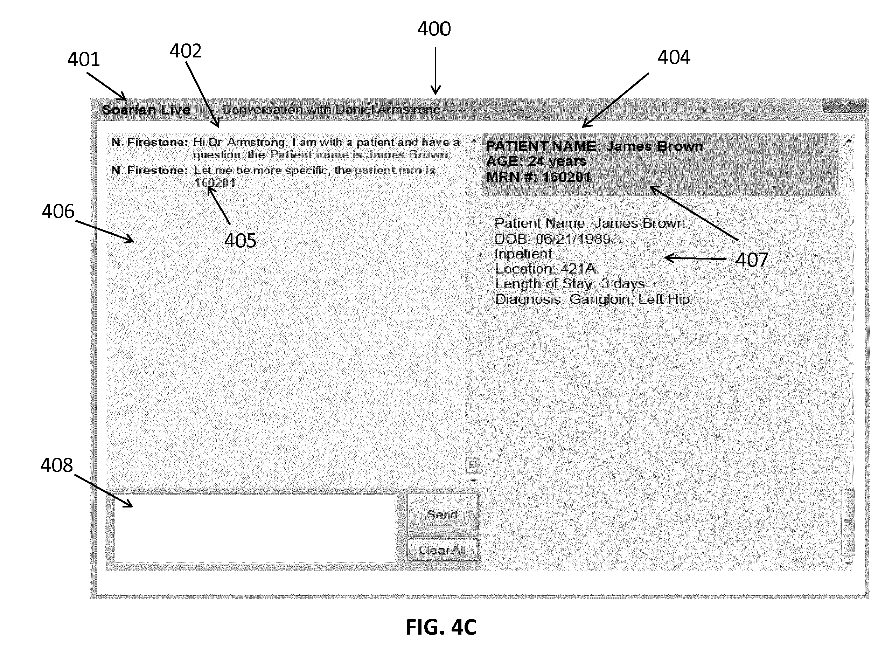

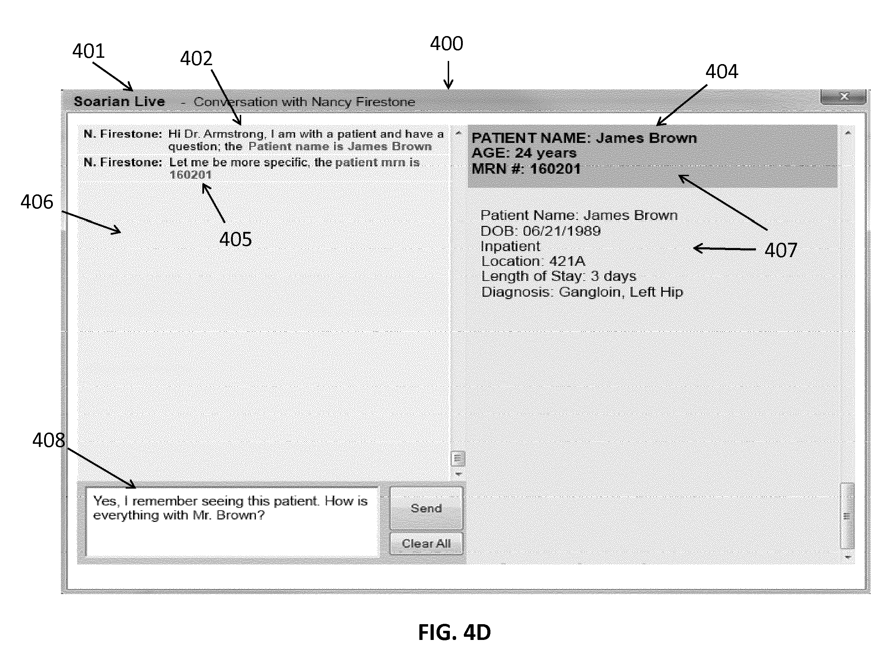

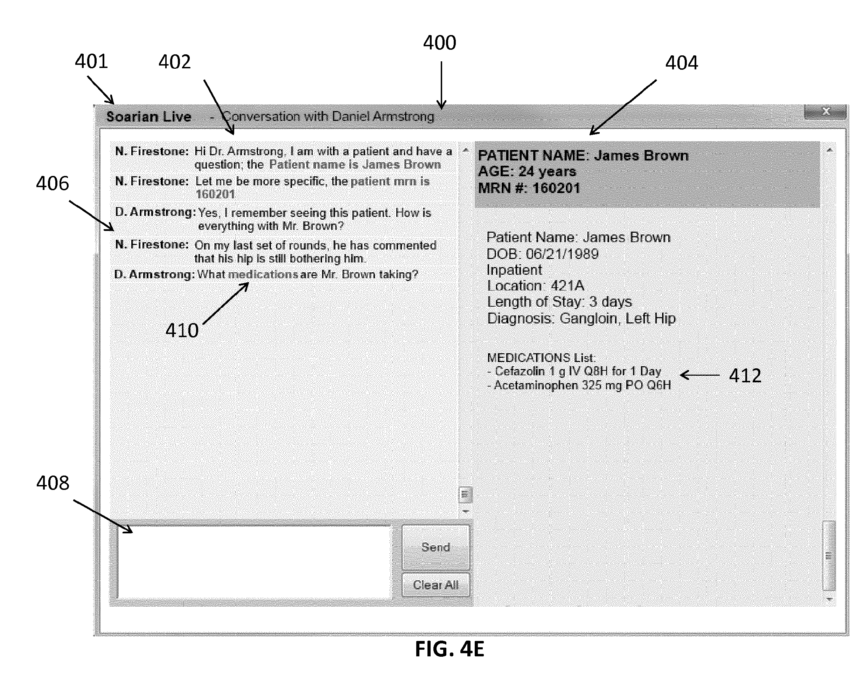

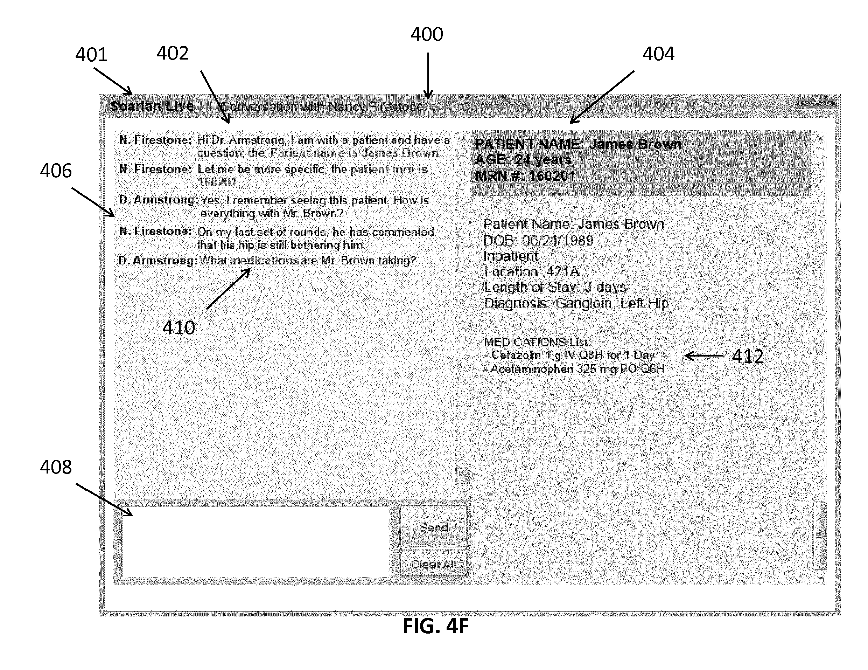

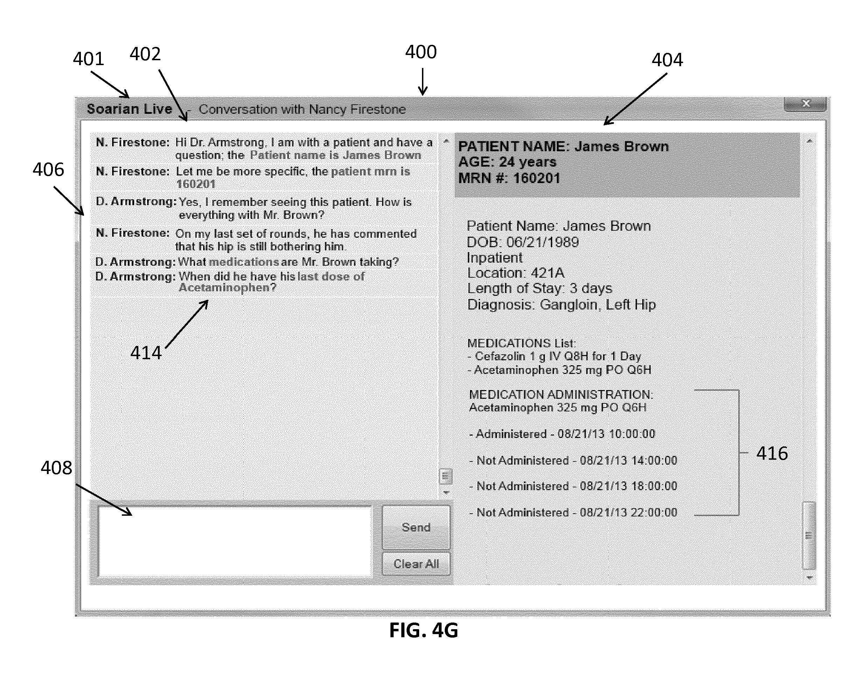

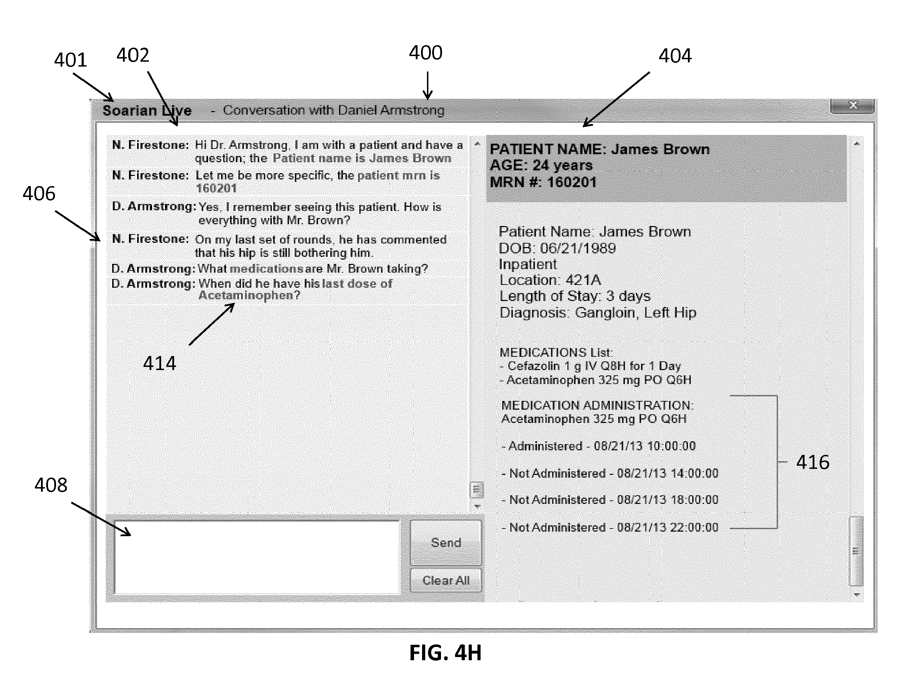

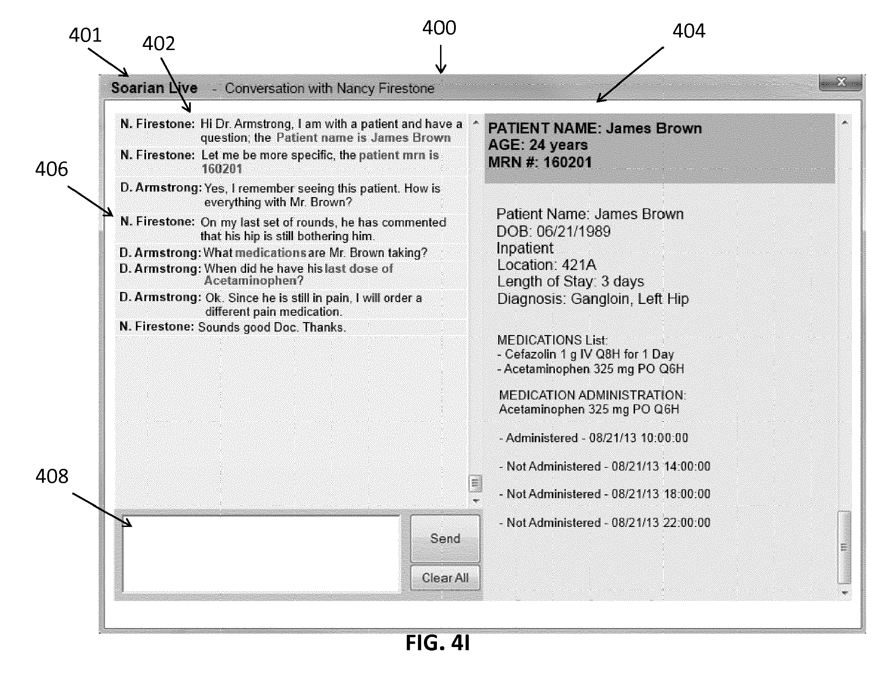

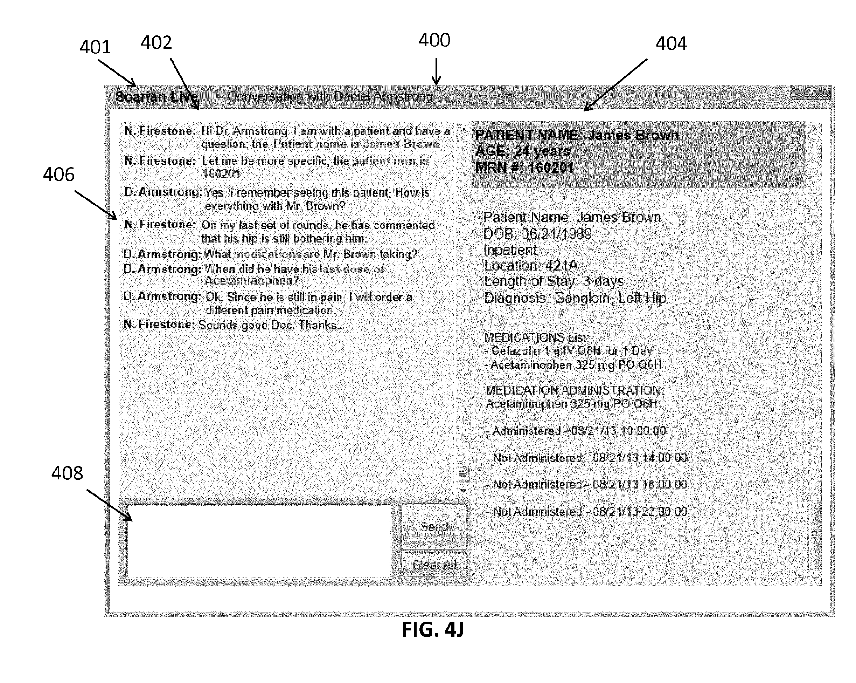

FIG. 4A through FIG. 4J are exemplary screen shots illustrating the simultaneous display of real time text communications and electronic patient medical records on an electronic device according to embodiments of the invention. The exemplary screen shots illustrate communications between a medical professional N. Firestone and Dr. Armstrong. As shown in FIG. 4A through 4J, the display 400 may include an area 401 identifying another user (e.g., Dr. Armstrong or N. Firestone) with whom the communication is presently being conducted. FIGs. 4A, 4C, 4E, 4H and 4J show the exemplary display 400 at the electronic device ofN. Firestone. FIGs. 4B, 4D, 4F, 4G and 4I show the exemplary display 400 at the electronic device of Dr. Armstrong. In some embodiments, communications may be conducted between more than users and displayed on electronic devices of more than two users. The display 400 may also include a text communication display area 402 and a medical context display area 404. The text communication display area 402 may also include a text communication history display area 406 and a present text communication area 408. The location, size and shape of each display area 402, 404, 406 and 408 are merely exemplary. Embodiments may include display areas having other sizes and shapes and at any location on displays of electronic devices.

As shown in FIG. 4A, text communication history display area 406 displays an exemplary original communication from the electronic device ofN. Firestone. The text communication recites "Hi Dr. Armstrong, I am with a patient and have a question; the patient name is James Brown." Accordingly, a receiving module, such as receiving module 202 (shown in FIG. 2) may receive the text communication from the electronic device (e.g., first electronic device 102 shown in FIG. 1) ofN. Firestone. As shown at text communication history display area 406, the original text communication may include the name of the patient, James Brown, as the patient identifier 403. In some embodiments, the terms "patient" or "name" may be an indicator that the patient identifier 403 is to follow within a predetermined number of characters. In some aspects, other terms may be used as an indicator that the patient identifier 403 is to follow. If the database interface 204 identifies a single patient within EMR database 106, database interface 204 may retrieve one or more portions of electronic patient medical records corresponding to a patient identifier.

Database interface module 204 may, however, identify multiple James Browns as patients having electronic medical records in database interface module 204. The identification of the multiple James Browns may then be displayed in medical context display area 404 on both the display 400 of N. Firestones electronic device (shown at FIG. 4A) and the display 400 of Dr. Armstrong's electronic device (shown at FIG. 4B). The display of the identification of multiple James Browns in medical context display area 404 may then prompt N. Firestone to input a second text communication in present text communication area 408 (shown in FIG. 4A) that includes a second patient identifier 405, such as the patient ID number 160201.

Upon receipt of the second text communication having the second patient identifier (patient ID number 160201) from the electronic device ofN. Firestone, database interface 204 may retrieve one or more portions of electronic patient medical records from EMR database 106 of the patient corresponding to the second patient identifier included in the second text communication received at receiving module 202. Transmitting module 206 may then transmit, via real time text communication server 108, portions of electronic patient medical records from EMR database 106 of the patient corresponding to patient ID number 160201. The second text communication having the second patient identifier 405 may then be displayed, via presentation modules 210, in the text communication history display area 406 at both the electronic device N. Firestone (shown in FIG. 4C) and the electronic device of Dr. Armstrong (shown in FIG. 4D). Further, portions 407 of the electronic patient medical records corresponding to patient ID number 160201 may also be displayed at the medical context display area 404 at both the electronic device N. Firestone (shown in FIG. 4C) and the electronic device of Dr. Armstrong (shown in FIG. 4D). In the embodiment shown in FIG. 4C, the initially displayed portions 407 of an identified patient's medical records may include basic information for a patient currently admitted to a hospital. Embodiments may, however, include initially displaying any type or amount of electronic patient medical records.

As shown in FIGs. 4D and 4E, a plurality of text communications pertaining to the patient may be transmitted between N. Firestone and Dr. Armstrong. Any of the communications transmitted between users may include a medical record data identifier. For example, as shown in text communication history display area 406 in FIG. 4E and FIG. 4F, the term "medications" 410 may be a medical record data identifier which causes additional medical record data 412 corresponding to the medical record data identifier 410 to be displayed in the medical context display area 404, such as a list of medications the patient is prescribed. Medical record data identifiers may include any number of characters (e.g., a word, a group of words, a part of a word, a symbol and a number).

Additional medical record data identifiers may be transmitted and displayed, thereby causing more medical record data to be displayed. For example, medical record data identifiers may include a current treatment of the patient, such as current or past medications. As shown in text communication history display area 406 in FIG. 4G, the term "Acetaminophen" 414 may be a medical record data identifier which causes additional medical record data 416 corresponding to "Acetaminophen" 414 to be displayed in the medical context display area 404. As shown in the medical context display area 416 of display 400 in FIG. 4G and FIG. 4H, a list of the acetaminophen that has been administered and the acetaminophen scheduled to be administered may be displayed.

The electronic patient medical records identifiers in the embodiments described herein are merely exemplary. Embodiments may include text communications having any type of electronic patient medical record identifiers associated with a patient. For example, the electronic patient medical records identifier may be part of a patient's anatomy (organ, appendage). Accordingly, the text communication and one or more additional portions of electronic medical record data corresponding to the part of the patient's anatomy may be simultaneously displayed.

As shown in FIG. 4I and FIG. 4J, additional communications may be transmitted and displayed on the display 400 of Dr. Armstrong's electronic device (FIG. 4I) and N. Firestone's electronic device (FIG. 4J) which do not cause any additional electronic patient medical records to be displayed.

Although the invention has been described with reference to exemplary embodiments, it is not limited thereto. Those skilled in the art will appreciate that numerous changes and modifications may be made to the preferred embodiments of the invention and that such changes and modifications may be made without departing from the true spirit of the invention. It is therefore intended that the appended claims be construed to cover all such equivalent variations as fall within the true spirit and scope of the invention.

## Claims

1. A computer implemented communication method between healthcare professionals, the method comprising:
receiving, from a first electronic device, a text communication having a patient identifier corresponding to a patient;
identifying, via a computer, the patient identifier of the text communication;
retrieving, via the computer, one or more portions of electronic patient medical records corresponding to the patient identifier;
transmitting, via a real time messaging server, the text communication and the one or more portions of the electronic patient medical records to a second electronic device; and
simultaneously displaying the text communication and the one or more portions of the electronic patient medical records at the second electronic device.

2. The communication method of claim 1, further comprising simultaneously displaying the text communication and the one or more portions of the electronic patient medical records at the first electronic device.

3. The communication method of claim 1, further comprising:
receiving a response text communication from the second electronic device; and
simultaneously displaying the response text communication and the one or more portions of electronic patient medical records at the first electronic device and the second electronic device.

4. The communication method of claim 3, wherein the text communication or the response text communication comprises a medical record identifier and the method further comprises simultaneously displaying the text communication or the response text communication and one or more additional portions of the electronic patient medical records corresponding to the medical record identifier.

5. The communication method of claim 4,
a) wherein the medical record identifier in the text communication or the response text communication is a part of a patient's anatomy and the method further comprises simultaneously displaying the text communication or the response text communication and the one or more additional portions of the electronic patient medical records corresponding to the part of the patient's anatomy, or
b) wherein the medical record identifier in the text communication or the response text communication is a current treatment of the patient and the method further comprises simultaneously displaying the text communication or the response text communication and the one or more additional portions of the electronic patient medical records corresponding to the current treatment of the patient, or
c) wherein the medical record identifier in the text communication or the response text communication comprises one or more medications prescribed to the patient and the method further comprises simultaneously displaying the text communication or the response text communication and the one or more additional portions of the electronic patient medical records corresponding to the one or more medications prescribed to the patient.

6. The communication method of claim 1, wherein simultaneously displaying the text communication and the one or more portions of the electronic patient medical records further comprises displaying the text communication in a first display area of a display and displaying the one or more portions of the electronic patient medical records in a second display area of the display.

7. The communication method of claim 1, further comprising:
identifying a plurality of patients each having one or more portions of electronic patient medical records that correspond to the patient identifier;
displaying the identification of the plurality of patients having one or more portions of electronic patient medical records; and
receiving, from the first electronic device, a second text communication having a second patient identifier corresponding to the patient, the second patient identifier being different from the first patient identifier.

8. A healthcare professional network communication system comprising:
a server comprising:
(i) a receiving module configured to receive a text communication from a first electronic device, the text communication having a patient identifier corresponding to a patient;
(ii) a database interface module configured to retrieve one or more portions of electronic patient medical records corresponding to the patient identifier; and
(iii) a transmitting module configured to transmit, via a real time messaging server, the text communication and the one or more portions of electronic patient medical records to a second electronic device; and
a first presentation module at the second electronic device configured to simultaneously present the text communication and the one or more portions of the electronic patient medical records on a display of the second electronic device.

9. The system according to claim 8, wherein
the receiving module is further configured to receive a response text communication from the second electronic device; and
the system further comprises:
a second presentation module at the first electronic device configured to present at least one of the text communication and the response text communication simultaneously with the one or more portions of the electronic patient medical records on a display of the first electronic device.

10. The system according to claim 9, wherein
the text communication or the response text communication comprises a medical record identifier,
the database interface module is further configured to retrieve one or more additional portions of the electronic patient medical records corresponding to the medical record identifier;
the transmitting module is further configured to transmit, via the real time messaging server, the text communication or the response text communication and the one or more additional portions of the electronic patient medical records between the first electronic device and the second electronic device,
the first presentation module at the second electronic device is further configured to present the text communication or the response text communication simultaneously with the one or more additional portions of the electronic patient medical records on the display of the second electronic device, and
the second presentation module at the first electronic device is further configured to present the text communication or the response text communication simultaneously with the one or more additional portions of the electronic patient medical records on the display of the first electronic device.

11. The system according to claim 8, wherein
the first presentation module at the second electronic device is further configured to simultaneously present: (i) the text communication at a first area of the display at the second electronic device; and (ii) the one or more portions of the electronic patient medical records at a second area of the display at the second electronic device.

12. The system according to claim 8, wherein
the database interface module is further configured to identify a plurality of patients each having one or more portions of electronic patient medical records that correspond to the patient identifier;
the first presentation module is further configured to display the identification of the plurality of patients each having one or more portions of electronic patient medical records;
the receiving module is further configured to receive a second text communication from the first electronic device having a second patient identifier corresponding to the patient, the second patient identifier being different from the first patient identifier;
the database interface module is configured to retrieve the one or more portions of the electronic patient medical records corresponding to the second patient identifier.

13. An article of manufacture for communicating between healthcare professionals, the article of manufacture comprising a non-transitory, tangible computer-readable medium holding computer-executable instructions for performing a method comprising:
receiving, from a first electronic device, a text communication having a patient identifier corresponding to a patient;
identifying, via a computer, the patient identifier of the communication;
retrieving, via the computer, one or more portions of electronic patient medical records corresponding to the patient identifier;
transmitting, via a real time messaging server, the text communication and the electronic medical record data to a second electronic device; and
simultaneously displaying the text communication and the one or more portions of the electronic patient medical records at the second electronic device.

14. The article of manufacture of claim 13,
a) further comprising instructions for simultaneously displaying the text communication and the one or more portions of the electronic patient medical records at the first electronic device, or
b) further comprising instructions for:
identifying a plurality of patients each having one or more portions of electronic patient medical records that correspond to the patient identifier;
displaying the identification of the plurality of patients having one or more portions of electronic patient medical records; and
receiving, from the first electronic device, a second text communication having a second patient identifier corresponding to the patient, the second patient identifier being different from the first patient identifier.

15. The article of manufacture of claim 13, further comprising instructions for:
receiving a response text communication from the second electronic device; and
simultaneously displaying the response text communication and the one or more portions of the electronic patient medical records at the first electronic device and the second electronic device.

16. The article of manufacture of claim 15, wherein the text communication or the response text communication comprises a medical record identifier and further comprising instructions for simultaneously displaying the text communication or the response text communication and one or more additional portions of the electronic patient medical records corresponding to the medical record identifier.

17. The article of manufacture of claim 13, wherein simultaneously displaying the text communication and the one or more portions of the electronic patient medical records further comprises displaying the text communication in a first display area of a display and displaying the one or more portions of the electronic patient medical records in a second display area of the display.
